(19) Europäisches Patentamt
European Patent Office
Office européen des brevets

(11) EP 3 015 473 B1

(12) # EUROPEAN PATENT SPECIFICATION

(45) Date of publication and mention of the grant of the patent:
**29.12.2021 Bulletin 2021/52**

(21) Application number: **14817084.8**

(22) Date of filing: **27.06.2014**

(51) Int Cl.:
***C07K 1/14*** *(2006.01)* ***C07K 7/06*** *(2006.01)*
***C07K 14/465*** *(2006.01)* ***B01D 15/12*** *(2006.01)*
***B01D 15/26*** *(2006.01)* ***B01D 15/36*** *(2006.01)*

(86) International application number:
**PCT/JP2014/067224**

(87) International publication number:
**WO 2014/208742 (31.12.2014 Gazette 2014/53)**

(54) **METHOD FOR PURIFYING OLIGOSACCHARIDE PEPTIDE**

VERFAHREN ZUR REINIGUNG VON OLIGOSACCHARID-PEPTIDEN

PROCÉDÉ DE PURIFICATION D'UN PEPTIDE À BASE D'OLIGOSACCHARIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2013 JP 2013136619**

(43) Date of publication of application:
**04.05.2016 Bulletin 2016/18**

(73) Proprietor: **Daiichi Sankyo Company, Limited**
**Tokyo 103-8426 (JP)**

(72) Inventor: **MIYAUCHI, Takahiro**
**Tokyo 134-8630 (JP)**

(74) Representative: **Arends, William Gerrit**
**Marks & Clerk LLP**
**15 Fetter Lane**
**London EC4A 1BW (GB)**

(56) References cited:

**WO-A1-2005/097155**
**JP-A- H06 245 784**
**JP-A- 2002 121 138**
**JP-A- 2011 162 762**
**JP-A- 2012 191 932**
**JP-A- 2012 224 577**
**US-A1- 2002 107 367**

- **MAMORU KOKETSU ET AL: "An efficient preparation and structural characterization of sialylglycopeptides from protease treated egg yolk", JOURNAL OF CARBOHYDRATE CHEMI, TAYLOR & FRANCIS, UK, vol. 14, no. 6, 1 August 1995 (1995-08-01) , pages 833-841, XP008182588, ISSN: 0732-8303, DOI: 10.1080/07328309508005379**
- **SEKO A ET AL: "Occurrence of a sialylglycopeptide and free sialylglycans in hen's egg yolk", BIOCHIMICA ET BIOPHYSICA ACTA (BBA) - GENERAL SUBJECTS, ELSEVIER, AMSTERDAM, NL, vol. 1335, no. 1-2, 17 April 1997 (1997-04-17), pages 23-32, XP004275961, ISSN: 0304-4165, DOI: 10.1016/S0304-4165(96)00118-3 [retrieved on 1997-10-15]**
- **ZENG Z ET AL: "Simultaneous determination of nine fluoroquinolones in egg white and egg yolk by liquid chromatography with fluorescence detection", JOURNAL OF CHROMATOGRAPHY B: BIOMEDICAL SCIENCES & APPLICATIONS, ELSEVIER, AMSTERDAM, NL, vol. 821, no. 2, 25 July 2005 (2005-07-25) , pages 202-209, XP027627249, ISSN: 1570-0232 [retrieved on 2005-07-25]**
- **SHUICHI SUGAWARA ET AL.: 'Sialyloligosaccharide Peptide (SGP) no Kenkyu Kaihatsu' ANNUAL REPORT OF THE NOGUCHI INSTITUTE vol. 54, 30 September 2011, pages 44 - 48, XP008182573**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KOKETSU, M. ET AL.: 'An efficient preparation and structural characterization of sialylglycopeptides from protease treated egg yolk.' J. CARBOHYDR. CHEM. vol. 14, no. 6, August 1995, pages 833 - 841, XP008182588
- None

## Description

### Technical Field

The present invention relates to a method for conveniently purifying an oligosaccharide peptide contained in an egg yolk component.

### Background Art

Sialyl glycopeptide (hereinafter, referred to as "SGP"), which is an oligosaccharide peptide contained in chicken egg yolk, contains a human-type oligosaccharide chain and is therefore useful as a raw material for pharmaceutical products.

The method disclosed in Patent Literature 1, etc. is known as a method for purifying SGP, which involves carrying out a series of extraction steps from a material containing delipidated chicken egg yolk in an aqueous solution to give an extract of the glycopeptide and subsequently a series of extraction steps for the precipitation of the extract of the glycopeptide using a water-soluble organic solvent such as ethanol, dissolving the obtained precipitates in water, and adsorbing SGP to a reverse-phase ODS resin (one type of resin for reverse-phase partition chromatography), followed by elution.

One technique known before Patent Literature 1 requires a series of concentration and desalting steps and is insufficient in terms of purity and yields (Patent Literature 2), and another one is based on purification by column chromatography, which is unsuitable for large-scale purification (Non Patent Literature 1).

### Citation List

#### Patent Literature

Patent Literature 1: International Publication No. WO2011/027868
Patent Literature 2: International Publication No. WO96/02255

#### Non Patent Literature

Non Patent Literature 1: Akira Seko, et al., Biochimica et Biophysica Acta, (1997), Vol. 1335, p. 23-32

Other methods are known for recovering sialylglycopeptides from egg yolk: M.KOKETSU ET AL: "An efficient preparation and structural characterization of sialylglycopeptides from protease treated egg yolk", JOURNAL OF CARBOHYDRATE CHEMISTRY, vol. 14, no.6, and A.SEKO ET AL: "Occurrence of a sialylglycopeptide and free sialylglycans in hen's egg yolk", BIOCHIMICA ET BIOPHYSICA ACTA, vol. 1335, no. 1-2, both disclose methods involving deproteinization of the solution followed by a chromatographic treatment.

### Summary of Invention

#### Technical Problem

The industrial-scale purification of highly pure SGP by heretofore known methods requires repeated extraction steps using a large amount of organic solvent and therefore requires unsafe operations and also a great deal of time, equipment, and effort.

#### Solution to Problem

The present inventor has conducted diligent studies on a method for purifying SGP and found that: when a deproteinizing agent, which is usually not used for protein or peptide purification, is added to a solution of an egg yolk component in water for removing an insoluble component, SGP remains in a dissolved portion, while other unnecessary proteins are removed due to the deproteinizing agent; and highly pure SGP can be purified at high yields by adsorbing the obtained dissolved portion to a synthetic adsorbent resin, followed by elution. The present inventor has conducted further studies and consequently completed the present invention.

The present invention provides the following aspects:

(1) A method for purifying sialyl glycopeptide, comprising mixing a synthetic silica-based deproteinizing agent with an aqueous solution containing avian egg yolk components including sialyl glycopeptide, thereby reducing the amount of proteins therein, and obtaining a dissolved portion from the mixture, wherein the sialyl glycopeptide remains in the dissolved portion.
(2) The method according to (1), wherein the synthetic silica-based deproteinizing agent is a synthetic silica gel or a synthetic silica colloid.
(3) The method according to (2), wherein the synthetic silica-based deproteinizing agent is a synthetic silica gel having a particle size of approximately 1 to 50 $\mu$m, a specific surface area of approximately 100 to 1500 $m^2$/g, a pore volume of approximately 0.5 to 3 ml/g, and an average pore size of approximately 1 to 50 nm.
(4) The method according to (1), wherein the aqueous solution containing avian egg yolk components including sialyl glycopeptide is a solution of chicken egg yolk or delipidated egg yolk in water.
(5) The method according to (1), wherein the aqueous solution containing avian egg yolk components including sialyl glycopeptide is a solution from which an insoluble component has been removed beforehand.
(6) The method according to (1), further comprising the step of recovering a fraction containing sialyl glycopeptide from the dissolved portion by column chromatography using a resin capable of separating sialyl glycopeptide.
(7) The method according to (6), wherein the resin capable of separating sialyl glycopeptide is a reverse-phase resin, a normal-phase resin, an ion-exchange resin, a gel filtration resin, or a synthetic adsorbent resin.
(8) The method according to (7), wherein the synthetic adsorbent resin is a styrene-divinylbenzene synthetic adsorbent resin, a modified styrene-divinylbenzene synthetic adsorbent resin, a methacrylic synthetic adsorbent resin, or a phenolic synthetic adsorbent resin.
(9) The method according to (8), wherein the synthetic adsorbent resin is a modified styrene-divinylbenzene synthetic adsorbent resin.

Advantageous Effects of Invention

The method of the present invention enables SGP to be purified in a short time and with less effort because the removal of protein components other than SGP from an egg yolk component requires only a step of adding a deproteinizing agent to and removing it from an aqueous solution of the component. In addition, this step does not employ an organic solvent and therefore, is safe and also eliminates the need of special equipment. Furthermore, a resin regeneration step by washing with an alkaline solution can be carried out when a synthetic adsorbent resin such as SEPABEADS SP207SS (Mitsubishi Chemical Corp.) is used as a resin for column packing used in the column chromatography step of recovering SGP from the deproteinized solution. This step enables the resin to be recycled and can further reduce cost. Moreover, the method of the present invention is provided as a method having less negative influence on the environment because the amount of waste liquid is small and a recyclable synthetic adsorbent resin can be selected.

Brief Description of Drawings

[Figure 1] Figure 1 is a chart showing HPLC measurement results of an SGP standard sample.
[Figure 2] Figure 2 is a chart showing LC/MS measurement results of the SGP standard sample.
[Figure 3] Figure 3 is a chart showing $^1$H-NMR measurement results of the SGP standard sample.
[Figure 4] Figure 4 is a chart showing HPLC measurement results of glycopeptide purified in Example 1.
[Figure 5] Figure 5 is a chart showing LC/MS measurement results of the glycopeptide purified in Example 1.
[Figure 6] Figure 6 is a chart showing $^1$H-NMR measurement results of the glycopeptide purified in Example 1.
[Figure 7] Figure 7 is a chart showing HPLC measurement results of glycopeptide purified in Example 2.
[Figure 8] Figure 8 is a graph indicating a relative protein concentration in a supernatant after treatment (bar graph) and the rate of SGP recovery (line graph), wherein the treatment was carried out with various synthetic silica gels (SYLORUTEs) as additives under each pH condition. The Y-axis depicts % both for the relative protein concentration (%) and for the rate of SGP recovery (%). The abscissa depicts the pH of a solution during the treatment with each additive. In the bar graph, the open bar represents the relative protein concentration after the 15-minute treatment, and the filled bar represents the relative protein concentration after the 60-minute treatment. In the line graph, ♦ represents the rate of SGP recovery after the 15-minute treatment, and ● represents the rate of SGP recovery after the 60-minute treatment.
[Figure 9] Figure 9 is a graph indicating a relative protein concentration in a supernatant after treatment (bar graph) and the rate of SGP recovery (line graph), wherein the treatment was carried out with various synthetic silica gels (Mizukasorbs) as additives under each pH condition. The Y-axis depicts % both for the relative protein concentration

(%) and for the rate of SGP recovery (%). The abscissa depicts the pH of a solution during the treatment with each additive. In the bar graph, the open bar represents the relative protein concentration after the 15-minute treatment, and the filled bar represents the relative protein concentration after the 60-minute treatment. In the line graph, ♦ represents the rate of SGP recovery after the 15-minute treatment, and ● represents the rate of SGP recovery after the 60-minute treatment.

[Figure 10] Figure 10 is a graph indicating a relative protein concentration in a supernatant after treatment (bar graph) and the rate of SGP recovery (line graph), wherein the treatment was carried out with various synthetic silica gels (Carplexes or Microd KM-386P) as additives under each pH condition. The Y-axis depicts % both for the relative protein concentration (%) and for the rate of SGP recovery (%) . The abscissa depicts the pH of a solution during the treatment with each additive. In the bar graph, the open bar represents the relative protein concentration after the 15-minute treatment, and the filled bar represents the relative protein concentration after the 60-minute treatment. In the line graph, ♦ represents the rate of SGP recovery after the 15-minute treatment, and ● represents the rate of SGP recovery after the 60-minute treatment.

[Figure 11] Figure 11 is a graph indicating a relative protein concentration in a supernatant after treatment (bar graph) and the rate of SGP recovery (line graph), wherein the treatment was carried out with various synthetic silica colloids (Coporocs) as additives under each pH condition. The Y-axis depicts % both for the relative protein concentration (%) and for the rate of SGP recovery (%). The abscissa depicts the pH of a solution during the treatment with each additive. In the bar graph, the open bar represents the relative protein concentration after the 15-minute treatment, and the filled bar represents the relative protein concentration after the 60-minute treatment. In the line graph, ♦ represents the rate of SGP recovery after the 15-minute treatment, and ● represents the rate of SGP recovery after the 60-minute treatment.

[Figure 12] Figure 12 is a graph indicating a relative protein concentration in a supernatant after treatment (bar graph) and the rate of SGP recovery (line graph), wherein the treatment was carried out with general filter aids such as Celite 545, Fibracel BH-40, or each Topco Perlite as additives under each pH condition. The Y-axis depicts % both for the relative protein concentration (%) and for the rate of SGP recovery (%). The abscissa depicts the pH of a solution during the treatment with each additive. In the bar graph, the open bar represents the relative protein concentration after the 15-minute treatment, and the filled bar represents the relative protein concentration after the 60-minute treatment. In the line graph, ♦ represents the rate of SGP recovery after the 15-minute treatment, and ● represents the rate of SGP recovery after the 60-minute treatment.

[Figure 13] Figure 13 is a graph indicating a relative protein concentration in a supernatant after treatment (bar graph) and the rate of SGP recovery (line graph), wherein the treatment was carried out with tannic acid and papain as additives under each pH condition. The Y-axis depicts % both for the relative protein concentration (%) and for the rate of SGP recovery (%). The abscissa depicts the pH of a solution during the treatment with each additive. In the bar graph, the open bar represents the relative protein concentration after the 15-minute treatment, and the filled bar represents the relative protein concentration after the 60-minute treatment (the oblique line bar for papain represents the relative protein concentration after the 20-hour treatment). In the line graph, ♦ represents the rate of SGP recovery after the 15-minute treatment, and ● represents the rate of SGP recovery after the 60-minute treatment (▲ for papain represents the rate of SGP recovery after the 20-hour treatment).

[Figure 14] Figure 14 is a chart showing HPLC measurement results of glycopeptide purified in Example 4.

[Figure 15] Figure 15 is a chart showing HPLC measurement results of glycopeptide purified in Example 5.

[Figure 16] Figure 16 is a chart showing HPLC measurement results of glycopeptide purified in Example 6.

[Figure 17] Figure 17 is a chart showing HPLC measurement results of glycopeptide purified in Example 7.

[Figure 18] Figure 18 is a chart showing HPLC measurement results of glycopeptide purified in Reference Example 1.

## Description of Embodiments

Hereinafter, the present invention will be described in detail.

The present invention provides a method for purifying sialyl glycopeptide, comprising mixing a synthetic silica-based deproteinizing agent with an aqueous solution containing avian egg yolk components including sialyl glycopeptide, thereby reducing the amount of proteins therein, and obtaining a dissolved portion from the mixture, wherein the sialyl glycopeptide remains in the dissolved portion.

<Mixing and separation>

In the present invention, the phrase "mixing" two or more materials means the step of contacting all of the materials with each other to cause the state where the respective components of the materials are uniformly distributed or dissolved. The mixing step is not particularly limited as long as it can achieve the mixed state mentioned above. Examples thereof can include shake stirring, stirring with a stirring bar, and stirring with a stirring blade. The time required

for such a step is not particularly limited and is, for example, 10 seconds or longer, preferably 5 minutes or longer, more preferably 15 minutes or longer. The temperature for this step is not particularly limited as long as the temperature neither deteriorates nor denatures the component contained in each material. The temperature is preferably 4°C to 40°C. In the present invention, the phrase "to obtain a dissolved portion" means that only a dissolved portion dissolved in a solvent is recovered by removing insoluble matter contained in a solution or a mixed solution. The step of separating the dissolved portion from insoluble matter can involve various methods known in the art. Typical examples of the separation step include filtration and centrifugation. A filter paper, a glass filter, a membrane filter, a filter cloth, or the like can be used as a filter for use in filtration. The mode of filtration can adopt natural filtration, suction filtration, pressure filtration, or the like. The type of the filter, the filtration mode, centrifugation conditions [the number of rotations, time, and temperature], etc., can be appropriately selected according to the state of the solution or the like subjected to separation. In order to improve the removal efficiency of insoluble matter from the solution, a filter aid such as Celite may be appropriately mixed in advance with the solution or the like prior to the separation step, and the separation step can then be carried out.

Various usually known filter aids having no deproteinizing effect can be adopted as the filter aid individually used.

In the present invention, the "dissolved portion" refers to a liquid sample that is a sample composed mainly of a liquid and is substantially free from insoluble matter as a result of removing the insoluble matter. Each component contained in the dissolved portion is present in a dissolved state in the liquid at least at the point in time when the dissolved portion has been obtained. The dissolved portion may generate insoluble matter during preservation due to change in liquid conditions (pH, temperature, etc.). Such insoluble matter can be removed by an ordinary method such as filtration so that the state substantially free from insoluble matter is restored.

<Sialyl glycopeptide>

In the present invention, the "sialyl glycopeptide" (hereinafter, referred to as "SGP") refers to an oligosaccharide peptide represented by the structural formula (I) and the sequence (II) given below in which carbon at the 1-position of GlcNAc at the reducing terminal of sialyl glycan composed of 11 sugar residues is bonded through a β-N-glycoside bond to a nitrogen atom derived from the side chain amide group of Asn at the 4-position of Lys-Val-Ala-Asn-Lys-Thr- (SEQ ID NO: 1).

[Formula 1]

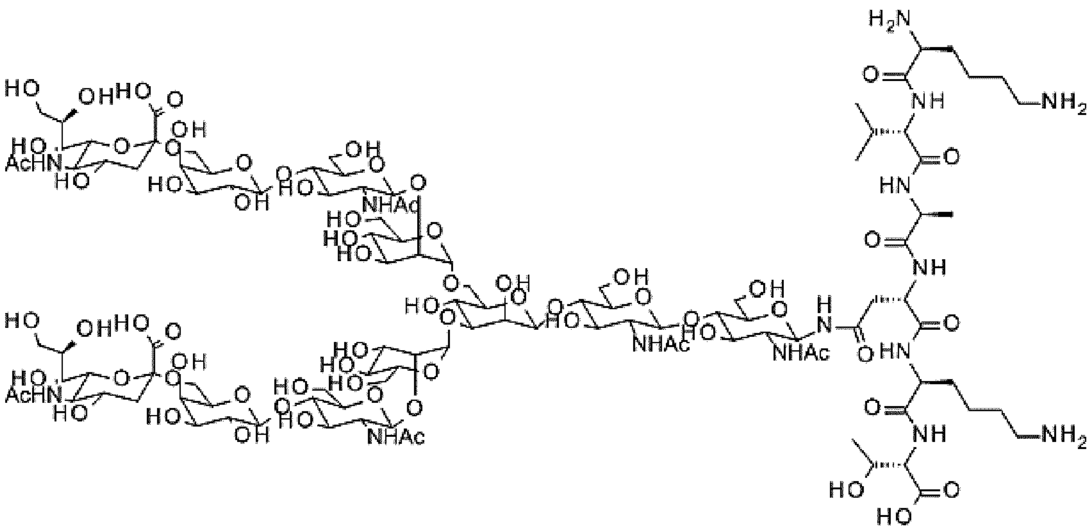

(I)

[Formula 2]

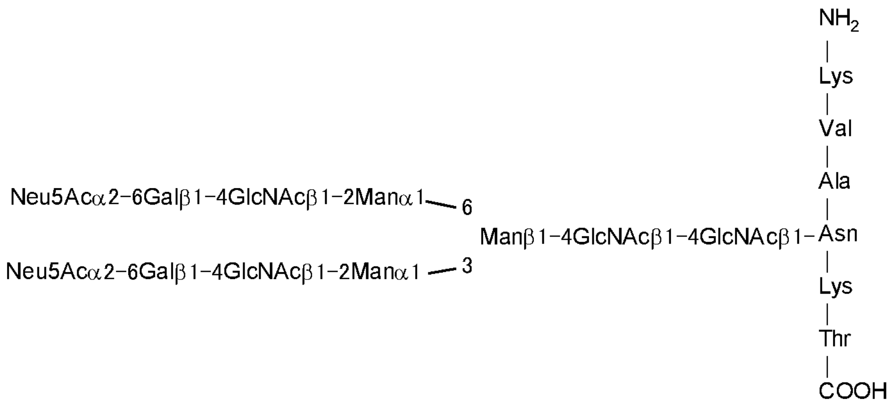

(II)

SGP is a glycopeptide contained in an avian egg yolk, and purified SGP is commercially available and can also be purchased. For example, the HPLC chart, LC/MS spectrum, and $^{1}$H-NMR spectrum (measured under conditions described in Example 1) of SGP manufactured by Tokyo Chemical Industry Co., Ltd. exhibit peaks as shown in Figures 1, 2, and 3, respectively. The obtained glycopeptide can therefore be identified as SGP with this commercially available SGP as a standard.

In the present invention, the "SGP product" refers to a product containing SGP as one of main constituents and is, for example, a research reagent, an industrial raw material, a pharmaceutical additive, or a food additive. In the case of producing the SGP product, SGP purified by the method of the present invention is processed into a state (freeze-dried powder, solution, etc.) suitable for a product form, included in a suitable container, and packaged to produce a product. SGP obtained by the purification method of the present invention is highly pure and therefore, is also useful as a raw material for such an SGP product.

SGP can be detected or quantified by HPLC or the like using a resin capable of separating SGP. Examples of conditions for such HPLC can include the following conditions:

[HPLC analysis conditions using reverse-phase column]

HPLC: 1260 Infinity LC (Agilent Technologies, Inc.)
Column: L-Column 2 ODS 3 μm ϕ3.0 × 50 mm (Chemical Evaluation and Research Institute, Japan)
Column temperature: 40°C
Mobile phase A: $H_2O$ solution containing 0.1% HCOOH (v/v)
Mobile phase B: MeCN solution containing 0.1% HCOOH (v/v)
Gradient (mobile phase B %): 0% (0 min), 10% (5 min), 30% (7 min), and 30% (8 min)
Flow rate: 0.6 ml/min
Detection wavelength: 210 nm

[HPLC analysis conditions using normal-phase column]

HPLC: 1260 Infinity LC (Agilent Technologies, Inc.)
Column: Inertsil Amide 3 μm ϕ3.0 × 75 mm (GL Sciences Inc.)
Column temperature: 40°C
Mobile phase A: $H_2O$ solution containing 0.1% HCOOH (v/v)

Mobile phase B: MeCN solution containing 0.1% HCOOH (v/v)
Gradient (mobile phase B %): 70% (0 min), 30% (6 min), 10% (6.01 min), and 10% (7.5 min)
Flow rate: 0.6 ml/min
Detection wavelength: 210 nm

The content of SGP contained in the solution can be calculated as a peak area value attributed to SGP in an HPLC chart obtained by subjecting the target solution to HPLC mentioned above. In the case of calculating, for example, the rate of recovery of SGP in the target solution with respect to the content of SGP in a starting material solution, the calculation can be conducted according to the following expression:

Rate of recovery (%) = (SGP peak area value of the target solution × Amount of the target solution) / (SGP peak area value of the starting material solution × Amount of the starting material solution) × 100

The purity of test SGP can be calculated as relative purity with the purity of standard SGP defined as 100%. For example, commercially available SGP manufactured by Tokyo Chemical Industry Co., Ltd. can be used as the standard SGP. As for a measurement method, accurately weighed test SGP is completely dissolved by the addition of a given amount of an aqueous solvent such as water, and the resulting test SGP solution is measured by HPLC under the conditions mentioned above. The peak area value of the test SGP in the obtained HPLC chart is measured. Likewise, the same step as above is also conducted as to the standard SGP to prepare a standard SGP solution having the same concentration as that of the test SGP. Then, the HPLC measurement is conducted under the same conditions as above. The purity of the test SGP can be calculated according to the following expression:

Purity of the test SGP (%) = (Peak area value of the test SGP / Peak area value of the standard SGP) × 100

<Solution containing egg yolk component>

SGP is known to be contained in avian egg yolk. The SGP contains a human-type sugar chain structure and is therefore less likely to cause allergic response even if humans ingest or take the SGP.

In the present invention, the "aqueous solution containing avian egg yolk components including sialyl glycopeptide" means a solution containing avian egg yolk components including sialyl glycopeptide mixed with an aqueous solvent. The origin of the egg is not particularly limited as long as the origin is a bird. Examples thereof can include chickens, quails, pigeons, ostriches, and crows. A chicken-derived egg is preferred. The egg yolk can be used in the method of the present invention directly as egg yolk separated from albumen from the whole egg, as dried egg yolk obtained by drying the egg yolk, as delipidated egg yolk obtained by the delipidation treatment of the egg yolk, or as an egg yolk powder or a delipidated egg yolk powder obtained by pulverizing the egg yolk or the delipidated egg yolk. The egg yolk powder, the delipidated egg yolk powder, or the like, derived from a chicken is commercially available and can be purchased (Yolk Protein H (Kewpie Corp.), Sunny Pro DF (Taiyo Kagaku Co., Ltd.), etc.).

Various aqueous solvents can be used as the solvent for dissolving or suspending the egg yolk component as long as the aqueous solvent is composed mainly of water and is substantially free from protein-denaturing components. The solvent is preferably water. A mixture of the egg yolk component such as egg yolk, delipidated egg yolk, or a powder thereof with the aqueous solvent may be used directly as the egg yolk component-containing solution for use in the deproteinization step. If necessary, only the dissolved portion from which insoluble matter has been separated may be used.

<Deproteinizing agent>

In the present invention, the "deproteinizing agent" means a material having the effect of reducing the amount of proteins in a protein-containing solution by the addition thereof to the solution (in the present specification, this effect is referred to as a "deproteinizing effect"). The mechanism underlying the deproteinizing effect of the deproteinizing

agent used in the present invention is not particularly limited unless the SGP content in the solution is reduced. Various deproteinizing agents, for example, a deproteinizing agent that adsorbs proteins, such as synthetic silica, a deproteinizing agent having a protein-coagulating effect, such as tannic acid, and a deproteinizing agent having a proteolytic effect, such as papain, can be adopted. The deproteinizing agent according to the present invention is a deproteinizing agent that adsorbs proteins, specifically a synthetic silica-based deproteinizing agent.

Various forms such as a synthetic silica gel in a powdery state or a synthetic silica colloid in a colloidal state can be adopted as the synthetic silica-based deproteinizing agent used in the present invention.

The synthetic silica gel is not particularly limited as long as the synthetic silica gel has a deproteinizing effect. Examples thereof include a synthetic silica gel having the following properties: an average particle size of approximately 1 to 50 (preferably, approximately 2 to 30) μm, a specific surface area of approximately 100 to 1500 (preferably, approximately 200 to 800) $m^2/g$, a pore volume of approximately 0.5 to 3 (preferably, approximately 1 to 2) ml/g, and an average pore size of approximately 1 to 50 (preferably, approximately 5 to 30) nm. Such a synthetic silica gel is commercially available as a filter aid. Specifically, for example, SYLOPUTE 202, SYLOPUTE 303, SYLOPUTE 403 (Fuji Silysia Chemical Ltd.), Carplex BS-303, Carplex BS-306 (DSL Japan Co., Ltd.), Microd KM-386P (KD corporation), Mizukasorb A751C, Mizukasorb C-1, or Mizukasorb C-6 (Mizusawa Industrial Chemicals, Ltd.) can be adopted.

Examples of the synthetic silica colloid include negatively charged and positively charged synthetic silica colloids. Various synthetic silica colloids can be applied to the present invention. In the case of adopting a negatively charged synthetic silica colloid, a favorable deproteinizing effect is obtained by adjusting the solution during the treatment to an acidic pH (pH of approximately 3 to 4). In the present invention, a weakly negatively charged synthetic silica colloid is preferred. Specifically, for example, Coporoc 200, Coporoc 300, or Coporoc 306 (Otsuka Foods Co., Ltd.) can be adopted as the synthetic silica colloid.

<Deproteinization step >

In the present invention, the mixing ratio for mixing the deproteinizing agent with the egg yolk component-containing solution differs depending on the type of the deproteinizing agent used. For example, the mixing ratio may be 0.1 to 50% (v/v or w/v) of the deproteinizing agent with respect to the amount of the solution. If necessary, the pH of the egg yolk component-containing solution may be appropriately adjusted before the mixing with the deproteinizing agent. Depending on the type of the deproteinizing agent, its deproteinizing effect or the rate of SGP recovery into a supernatant may vary in a pH-dependent manner. The purification efficiency of SGP can therefore be improved by selecting suitable pH according to the deproteinizing agent used. Since each SYLOPUTE, each Mizukasorb, each Carplex, or Microd KM-386P, for example, tends to have a high deproteinizing effect and also a high rate of SGP recovery into a supernatant at a weakly acidic pH on the order of 3 to 6, it is preferred for improving the purification efficiency of SGP to adjust the egg yolk component-containing aqueous solution to be weakly acidic.

The duration for the mixing of the deproteinizing agent with the egg yolk component-containing solution is not particularly limited, provided that the mixing thereby reduces the amount of proteins in the mixture. In the case of using an additive having a proteolytic effect as the deproteinizing agent, treatment for a long time may reduce the SGP content in the solution following treatment due to the degradation of SGP, etc. For this reason, the mixing time in the case of using the deproteinizing agent having a proteolytic effect is usually 5 hours or shorter, preferably 3 hours or shorter, more preferably 2 hours or shorter, most preferably 1 hour or shorter.

After the mixing of the deproteinizing agent with the egg yolk component solution, insoluble matter is separated to obtain a dissolved portion.

The content of SGP in the dissolved portion obtained by the separation step mentioned above can be confirmed by a method such as HPLC. The protein content in the dissolved portion can be identified or determined by various methods known in the art. For example, SDS-PAGE can be used as the method for identifying proteins. Alternatively, an ultraviolet absorption method (absorbance: 280 nm) or a Bradford method can be adopted as the method for quantifying proteins. The deproteinization step mentioned above may be repeated or a plurality of deproteinization steps may be appropriately combined, for example, if it is found in such protein identification or quantification that proteins are not sufficiently removed from the mixture.

<Purification of SGP from deproteinized solution>

SGP can be purified by use of various column packing materials from the deproteinized solution obtained through the deproteinization step mentioned above. Examples of the column packing material for use in such purification can include a synthetic adsorbent resin, a reverse-phase resin, a normal-phase resin, an ion-exchange resin, and a gel filtration resin. The reverse-phase resin refers to a resin having a hydrophobic functional group such as a butyl group (C4), an octyl group (C8), an octadecyl group (C18), a triacontyl group (C30), or a phenyl group added to a base material of a silica gel. Particularly, the purification of SGP using a reverse-phase resin adding an octadecyl group (Wakogel

100C18 (Wako Pure Chemical Industries, Ltd.)) is described in Patent Literature 1. Also, the purification of SGP using an ion-exchange resin Toyopearl DEAE-650M (Tosoh Corp.), CM-Sephadex C-25 (GE Healthcare UK Ltd.), or Dowex 50Wx2 (The Dow Chemical Company) and the purification of SGP using a gel filtration resin Sephadex G-50 (GE Healthcare UK Ltd.) or Sephadex G-25 (GE Healthcare UK Ltd.) are each described in Non Patent Literature 1. SGP can be appropriately purified with reference to conditions described in these literatures.

<Synthetic adsorbent resin>

The present invention provides a method for purifying SGP using a synthetic adsorbent resin as the column packing material in the purification step. The synthetic adsorbent resin refers to a packing material composed of a porous polymer having a cross-linked structure. Examples thereof include a styrene-divinylbenzene synthetic adsorbent, a modified styrene-divinylbenzene synthetic adsorbent resin, a methacrylic synthetic adsorbent resin, and a phenolic synthetic adsorbent resin, depending on the type of the polymer. A packing material, such as a reverse-phase ODS resin, known to be usable in the purification of SGP has a silica gel as a base material and therefore, does not permit washing with an alkaline solution. This resin cannot therefore be recycled because impurities are insufficiently removed by mere washing with an organic solvent. Use of the synthetic adsorbent resin as the column packing material, however, permits washing with an organic solvent as well as resin regeneration by washing with an alkaline solution. The synthetic adsorbent resin can therefore sufficiently remove impurities and can also be recycled. Various synthetic adsorbent resins capable of adsorbing SGP can be adopted as such a synthetic adsorbent resin. The synthetic adsorbent resin is preferably a styrene-divinylbenzene synthetic adsorbent resin, more preferably a modified styrene-divinylbenzene synthetic adsorbent resin. Specific examples of products can include SEPABEADS SP207SS (Mitsubishi Chemical Corp.).

According to the method above, SGP can be purified by allowing the deproteinized solution obtained by the step mentioned above to pass through a column packed with the column packing material, washing, if necessary, the column with a solvent suitable for the separation mode of chromatography, eluting SGP using mobile phases appropriate for the separation mode of chromatography, and recovering a fraction containing SGP in the eluate. Prior to this purification step, if necessary, conditions for the deproteinized solution may be appropriately adjusted according to the column packing material used or the mode of chromatography. If insoluble matter is generated due to this change in the solution conditions, the solution is appropriately mixed with a filter aid such as Celite and then used in the step of recovering only the dissolved portion. SGP contained in the eluted fraction can also be monitored under the HPLC conditions mentioned above, or the eluted fraction of SGP can also be identified from the elution time provided that the elution is carried out under the same conditions as previous conditions. Such purification conditions can be appropriately set according to the type and properties of the adopted column packing material.

In the case of using, for example, SEPABEADS SP207SS as the column packing material, the deproteinized solution is adjusted to a neutral to alkaline pH of 7 or higher (preferably a pH around 9), and only the dissolved portion from which insoluble matter has been removed is allowed to pass through a column packed with SEPABEADS SP207SS. The column can be washed with water, followed by elution with a concentration gradient of water to 2% acetone in water.

The obtained purified SGP solution may be cryopreserved as it is, or may be concentrated or freeze-dried, if necessary, and preserved.

Examples

Hereinafter, the present invention will be described specifically with reference to Examples. Examples disclosed herein are given merely for the purpose of illustrating the embodiments of the present invention and are not intended to limit the present invention.

<Example 1. Purification of SGP using synthetic silica gel - 1>

To 500 g of a delipidated egg yolk powder (Kewpie Corp.), 5 L of water was added, and the mixture was well stirred for 15 minutes and then filtered using a filter press (Yabuta Kikai Co., Ltd.).

To the obtained mixture, 250 g of SYLOPUTE 403 (Fuji Silysia Chemical Ltd.) was added, and the mixture was well stirred for 15 minutes and then filtered using a filter press (Yabuta Kikai Co., Ltd.).

The obtained filtrate was adjusted to pH 9 with 1 M NaOH and well stirred. The resulting filtrate was allowed to stand for 30 minutes. Then, 100 g of Celite 545 (Imerys Minerals California, Inc.) was added thereto, and the mixture was filtered using a filter press (Yabuta Kikai Co., Ltd.).

A column was packed with 207 mL (35ϕ x 215 mm) of a synthetic adsorbent resin SEPABEADS SP207SS (Mitsubishi Chemical Corp.), and the resin was washed with acetone and subsequently with water and then equilibrated with 20 mM $Na_2HPO_4$.

The filtrate obtained above was applied to the equilibrated resin at a flow rate of 50 mL/min. The filtrate-applied

resin was washed with 2 L of water at a flow rate of 50 mL/min and then developed with 2% acetone in water (v/v) at a flow rate of 25 mL/min, and the eluate was fractionated into each 25-mL portion (eluted fractions 1 to 40).

The eluted fractions 18 to 26 were combined and freeze-dried to obtain 764.4 mg of glycopeptide.

The obtained glycopeptide and a standard sample (SGP manufactured by Tokyo Chemical Industry Co., Ltd.) were subjected to $^1$H-NMR measurement, LC/MS measurement, and HPLC analysis under conditions given below. The HPLC, LC/MS, and $^1$H-NMR measurement results of the standard sample are shown in Figures 1, 2, and 3, respectively. The HPLC, LC/MS, and $^1$H-NMR measurement results of the obtained glycopeptide are shown in Figures 4, 5, and 6, respectively.

The obtained glycopeptide was confirmed to be SGP represented by the formulas (I) and (II) because a mass spectrum and a $^1$H-NMR spectrum similar to those of the standard sample were obtained as compared with the standard sample in the $^1$H-NMR measurement and the LC/MS measurement. In the HPLC results, the obtained SGP had a purity of 104% from the ratio of relative peak area values attributed to SGP between the obtained SGP and the standard sample.

[HPLC measurement]

HPLC: 1260 Infinity LC (Agilent Technologies, Inc.)
Column: L-Column 2 ODS 3 μm ϕ3.0 × 50 mm (Chemical Evaluation and Research Institute, Japan)
Column temperature: 40°C
Mobile phase A: $H_2O$ solution containing 0.1% HCOOH (v/v)
Mobile phase B: MeCN solution containing 0.1% HCOOH (v/v)
Gradient (mobile phase B %): 0% (0 min), 10% (5 min), 30% (7 min), and 30% (8 min)
Flow rate: 0.6 ml/min
Detection wavelength: 210 nm

[LC/MS measurement]

MS: 6130 Quadrupole LC/MS (Agilent Technologies, Inc.)
Ionization: ESI
Mode: Positive
HPLC: 1260 Infinity LC (Agilent Technologies, Inc.)
Column: L-Column 2 ODS 3 μm ϕ3.0 × 50 mm (Chemical Evaluation and Research Institute, Japan)
Column temperature: 40°C
Mobile phase A: $H_2O$ solution containing 0.1% HCOOH (v/v)
Mobile phase B: MeCN solution containing 0.1% HCOOH (v/v)
Gradient (mobile phase B %): 0% (0 min), 10% (5 min), 30% (7 min), and 30% (8 min)
Flow rate: 0.6 ml/min
Detection wavelength: 210 nm

[$^1$H-NMR measurement]

NMR: AVANCE 500 (500 MHz, Bruker BioSpin K.K.)
Solvent: deuterium oxide + 0.1% TMSP (Euriso-Top)

<Example 2. Purification of SGP using synthetic silica gel - 2>

Glycopeptide was purified from a delipidated egg yolk powder by the same method as in Example 1. The column eluted fractions 20 to 30 were combined and freeze-dried to obtain 694.1 mg of glycopeptide.

The obtained glycopeptide was confirmed to be SGP by comparison with the standard sample in $^1$H-NMR measurement and LC/MS measurement.

The obtained SGP was subjected to HPLC analysis under the same conditions as in Example 1 (Figure 7). The purity was calculated in the same way as in Example 1 from the HPLC results. Consequently, the obtained SGP had a purity of 102% from the ratio of relative peak area values attributed to SGP between the obtained SGP and the

standard sample.

<Example 3. Study on application of diverse deproteinizing agents to SGP purification>

Next, various deproteinizing agents, various general filter aids, and various additives were studied for their applicability to SGP purification.

For the purification method of the present invention, it is important that impurities, particularly, unnecessary proteins, are removed from a solution that is subjected to column chromatography, while SGP remains in the solution. The study was therefore made using, as indexes, the content of proteins in a solution after treatment with a deproteinizing agent or the like and the rate of SGP recovery.

Testing for deproteinizing ability in delipidated egg yolk powder and rate of SGP recovery

Synthetic silica gels, synthetic silica colloids, general filter aids, an additive having a protein-coagulating effect, and an additive having a proteolytic effect were evaluated for their protein removal performance for proteins in a delipidated egg yolk powder and also evaluated for the rate of recovery of SGP in the delipidated egg yolk powder.

[Additive]

The synthetic silica gels used were SYLOPUTE 202 (Fuji Silysia Chemical Ltd.), SYLOPUTE 303 (Fuji Silysia Chemical Ltd.), SYLOPUTE 403 (Fuji Silysia Chemical Ltd.), Mizukasorb A751C (Mizusawa Industrial Chemicals, Ltd.), Mizukasorb C1 (Mizusawa Industrial Chemicals, Ltd.), Mizukasorb C6 (Mizusawa Industrial Chemicals, Ltd.), Carplex BS-303 (DSL Japan Co., Ltd.), Carplex BS-306 (DSL Japan Co., Ltd.), and Microd KM-386P (KD Corporation).

The synthetic silica colloids used were Coporoc 200 (Otsuka Foods Co., Ltd.), Coporoc 300 (Otsuka Foods Co., Ltd.), and Coporoc 306 (Otsuka Foods Co., Ltd.).

The general filter aids used were Celite 545 (Imerys Minerals California, Inc.), Fibracel BH-40 (Imerys Minerals California, Inc.), Topco Perlite No. 31 (Toko Perlite Kogyo Ltd.), and Topco Perlite No. 31 (Toko Perlite Kogyo Ltd.).

The additive having a protein-coagulating effect used was tannic acid (Tokyo Chemical Industry Co., Ltd.). The additive having a proteolytic effect used was papain (Wako Pure Chemical Industries, Ltd.).

[Method]

To a delipidated egg yolk powder (Kewpie Corp.), a 10-fold amount of water was added (concentration: 100 mg/mL), and the mixture was well stirred for 15 minutes and then filtered using FILTER PAPER No. 2 (Toyo Roshi Kaisha, Ltd.). The obtained solution of delipidated egg yolk in water was adjusted to pH 3, pH 4, pH 5, pH 6, pH 7, or pH 8, and 10 mL of each solution was then dispensed.

Each synthetic silica gel or each general filter aid was added at 5% (w/v) to the dispensed solution and contacted therewith by stirring at room temperature for 15 minutes and 60 minutes.

Each silica colloid was added at 5% (v/v) to the dispensed solution and contacted therewith by stirring at room temperature for 15 minutes and 60 minutes.

Tannic acid was added at 1% (w/v) to the dispensed solution and contacted therewith by stirring at room temperature for 15 minutes and 60 minutes.

Papain was added at 1% (w/v) to the dispensed solution and contacted therewith by stirring at room temperature for 15 minutes and 60 minutes and by stirring at 50°C for 20 hours.

All stirring procedures for the experiment were carried out using a reciprocal shaker.

Each treated sample was centrifuged at 10,000 rpm for 5 minutes. The obtained supernatant was recovered and both the protein content and the rate of SGP recovery were measured by methods given below. The results are shown in Figures 8 to 13.

a) Measurement of protein content by Bradford method [Bradford, M. M., Anal. Biochem. 72: 248-254 (1976)]

A standard and the supernatant were each dispensed (15 μL), and 1.5 mL of a chromogenic solution was added to each dispensed solution. After stirring, the mixture was kept at room temperature for 10 minutes or longer and assayed. The chromogenic solution used was Coomassie Protein Assay Reagent (Thermo Fisher Scientific, Inc.). The standard used was BSA (bovine serum albumin, Thermo Fisher Scientific, Inc.). The measurement wavelength was set to absorbance of 595 nm. The results were indicated in the drawings by relative protein concentration (%) with the protein concentration in the solution of delipidated egg yolk in water defined as 100%.

b) Measurement of rate of SGP recovery by HPLC.

The peak area value of SGP was measured by analysis under the HPLC conditions of Example 1. Likewise, 5 μL each of the solution of delipidated egg yolk in water and each supernatant was injected to an HPLC unit and analyzed under the HPLC conditions of Example 1 to measure the peak area value of SGP. The rate of SGP recovery was also indicated in the drawings by relative peak area value (%) with the peak area value of SGP in the solution of delipidated egg yolk in water defined as 100%.

[Results]

As shown in Figures 8 to 10, the solution treated with each synthetic silica gel was found to have a high deproteinizing effect that resulted in a low protein concentration. On the other hand, this treated solution had a high rate of SGP recovery, indicating that SGP was dissolved in the solution without being adsorbed to the synthetic silica. These effects are slightly influenced by the pH of the solution during the treatment, and the deproteinizing effect and the rate of SGP recovery both tend to be high under conditions closer to an acidic pH. It is however suggested that highly pure SGP can be purified from the treated solution under any of the conditions.

In the case of treatment with each synthetic silica colloid, as shown in Figure 11, different tendencies were found depending on the type. Coporoc 200 was found to have a definite deproteinizing effect and rate of SGP recovery, suggesting that highly pure SGP can be purified at any of the pHs. By contrast, Coporoc 300 and Coporoc 306 were found to have a sufficient deproteinizing effect and high rate of SGP recovery at a pH around 3 or 4, but resulted in a high protein concentration in the solution after the treatment at a pH of 6 or higher. The colloidal synthetic silica was also demonstrated to have a high deproteinizing effect.

As shown in Figure 12, the general filter aids such as Celite 545, Fibracel BH-40, and each Perlite were demonstrated to have no deproteinizing effect. This indicated that among the general silica filter aids, natural silica such as Celite has no deproteinizing effect, while use of synthetic silica produces a deproteinizing effect, which is an important property for the additive used in the method of the present invention.

As for a material known to have a deproteinizing effect except for synthetic silica, i.e., tannic acid or papain, as shown in Figure 13, tannic acid was found to have a definite deproteinizing effect and rate of SGP recovery, regardless of pH. In the case of treatment with papain, both the deproteinizing effect and the rate of SGP recovery vary largely depending on pH, and conditions closer to an acidic pH were found to be suitable. Since the 20-hour papain treatment reduces the rate of SGP recovery, the treatment time should be set to a few hours or shorter, and a treatment time on the order of 15 minutes to 60 minutes seems to be suitable.

<Example 4. Purification of SGP using synthetic silica gel - 3>

To 500 g of delipidated egg yolk powder (Kewpie Corp.), 5 L of water was added, and the mixture was well stirred for 15 minutes and then filtered using a filter press (Yabuta Kikai Co., Ltd.).

To the obtained water extracts, 250 g of Mizukasorb A751C (Mizusawa Industrial Chemicals, Ltd.) was added, and the mixture was well stirred for 15 minutes and then filtered using a filter press (Yabuta Kikai Co., Ltd.).

The obtained filtrate was adjusted to pH 9 with 1 M NaOH and well stirred. The resulting filtrate was allowed to stand for 30 minutes. Then, 100 g of Celite 545 (Imerys Minerals California, Inc.) was added thereto, and the mixture was filtered using a filter press (Yabuta Kikai Co., Ltd.).

A column was packed with 207 mL (35ϕ x 215 mm) of synthetic adsorbent resin SEPABEADS SP207SS (Mitsubishi Chemical Corp.), and the resin was washed with acetone and subsequently with water and then equilibrated with 20 mM $Na_2HPO_4$.

The filtrate obtained above was applied to the equilibrated resin at a flow rate of 50 mL/min. The filtrate-applied resin was washed with 2 L of water at a flow rate of 50 mL/min and then developed with 2% acetone in water (v/v) at a flow rate of 25 mL/min, and the eluate was fractionated on a 25-mL basis (eluted fractions 1 to 40).

The eluted fractions 17 to 22 were combined and freeze-dried to obtain 733.8 mg of glycopeptide.

The obtained glycopeptide was confirmed to be SGP by comparison with the standard sample in $^1$H-NMR measurement and LC/MS measurement. The obtained SGP was subjected to HPLC analysis under the same conditions as in Example 1 to calculate the purity (Figure 14, purity: 98%).

<Example 5. Purification of SGP using synthetic silica gel - 4>

Glycopeptide was purified from a delipidated egg yolk powder by the same method as in Example 4. The column eluted fractions 17 to 22 were combined and freeze-dried to obtain 784.1 mg of glycopeptide.

The obtained glycopeptide was confirmed to be SGP by comparison with the standard sample in $^1$H-NMR

measurement and LC/MS measurement. The obtained SGP was subjected to HPLC analysis under the same conditions as in Example 1 to calculate the purity (Figure 15, purity: 99%).

<Example 6. Purification of SGP using reverse-phase ODS resin>

To 500 g of a delipidated egg yolk powder (Kewpie Corp.), 5 L of water was added, and the mixture was well stirred for 15 minutes and then filtered using a filter press (Yabuta Kikai Co., Ltd.).

To the obtained water extracts, 250 g of Mizukasorb A751C (Mizusawa Industrial Chemicals, Ltd.) was added, and the mixture was well stirred for 15 minutes and then filtered using a filter press (Yabuta Kikai Co., Ltd.).

The obtained filtrate was adjusted to pH 6.5 with 1 M NaOH and well stirred. The resulting filtrate was allowed to stand for 30 minutes. Then, 100 g of Celite 545 (Imerys Minerals California, Inc.) was added thereto, and the mixture was filtered using a filter press (Yabuta Kikai Co., Ltd.).

A column was packed with 192 mL (35ϕ x 200 mm) of reverse-phase ODS resin Wakogel 100C18 (Wako Pure Chemical Industries, Ltd.), and the resin was washed with acetonitrile and subsequently with water and then equilibrated with 20 mM $HCOONH_4$.

The filtrate obtained above was applied to the equilibrated resin at a flow rate of 50 mL/min. The filtrate-applied resin was washed with 2 L of water at a flow rate of 50 mL/min and then developed with 1% acetonitrile in water (v/v) at a flow rate of 25 mL/min, and the eluate was fractionated on a 25-mL basis (eluted fractions 1 to 14). The resin was further developed with 2% acetonitrile in water (v/v) at a flow rate of 25 mL/min, and the eluate was fractionated on a 25-mL basis (eluted fractions 15 or 40).

The eluted fractions 8 to 24 were combined and freeze-dried to obtain 565.6 mg of glycopeptide.

The obtained glycopeptide was confirmed to be SGP by comparison with the standard sample in $^1$H-NMR measurement and LC/MS measurement. The obtained SGP was subjected to HPLC analysis under the same conditions as in Example 1 to calculate the purity (Figure 16, purity: 98%).

<Example 7. Purification of SGP using dried egg yolk powder>

To 500 g of dried egg yolk powder No. 1 (Kewpie Corp.), 5 L of water was added, and the mixture was well stirred for 15 minutes and then filtered using a suction filtration can (Sugiyama-Gen Co., Ltd.) after addition of 1 kg of Celite 545 (Imerys Minerals California, Inc.).

To the obtained water extracts, 250 g of Mizukasorb A751C (Mizusawa Industrial Chemicals, Ltd.) was added, and the mixture was well stirred for 15 minutes and then filtered using a suction filtration can (Sugiyama-Gen Co., Ltd.).

The obtained filtrate was adjusted to pH 9 with 1 M NaOH and well stirred. The resulting filtrate was allowed to stand for 30 minutes. Then, 100 g of Celite 545 (Imerys Minerals California, Inc.) was added thereto, and the mixture was filtered using a suction filtration can (Sugiyama-Gen Co., Ltd.).

A column was packed with 207 mL (35ϕ x 215 mm) of synthetic adsorbent resin SEPABEADS SP207SS (Mitsubishi Chemical Corp.), and the resin was washed with acetone and subsequently with water and then equilibrated with 20 mM $Na_2HPO_4$.

The filtrate obtained above was applied to the equilibrated resin at a flow rate of 50 mL/min. The filtrate-applied resin was washed with 2 L of water at a flow rate of 50 mL/min and then developed with 2% acetone in water (v/v) at a flow rate of 25 mL/min, and the eluate was fractionated on a 25-mL basis (eluted fractions 1 to 40).

The eluted fractions 18 to 21 were combined and freeze-dried to obtain 212.2 mg of glycopeptide.

The obtained glycopeptide was confirmed to be SGP by comparison with the standard sample in $^1$H-NMR measurement and LC/MS measurement. The obtained SGP was subjected to HPLC analysis under the same conditions as in Example 1 to calculate the purity (Figure 17, purity: 93%).

<Reference Example 1. Purification of SGP using general filter aid>

Column eluted fractions 14 or 24 were combined and freeze-dried to obtain 955.0 mg of glycopeptide in the same way as in Example 4 except that 250 g of a general filter aid Celite 545 (Imerys Minerals California, Inc.) was used instead of the synthetic silica gel (Mizukasorb A751C).

The obtained glycopeptide was confirmed to be SGP by comparison with the standard sample in $^1$H-NMR measurement and LC/MS measurement. The obtained SGP was subjected to HPLC analysis under the same conditions as in Example 1 to calculate the purity (Figure 18, purity: 83%).

Celite 545 has no deproteinizing effect, as shown in Example 3, and therefore failed to sufficiently remove unnecessary proteins in the solution in this Reference Example. As a result, the obtained SGP had an insufficient purity even after the separation step using the synthetic adsorbent, as compared with other Examples using the deproteinizing agent. This also indicated that for obtaining highly pure SGP, it is important to sufficiently remove unnecessary proteins in a dissolved portion by mixing and separating a slurry of an egg yolk component-containing solution and a filter aid having a deproteinizing effect.

## Claims

1. A method for purifying sialyl glycopeptide, comprising mixing a synthetic silica-based deproteinizing agent with an aqueous solution containing avian egg yolk components including sialyl glycopeptide, thereby reducing the amount of proteins therein, and obtaining a dissolved portion from the mixture, wherein the sialyl glycopeptide remains in the dissolved portion.

2. The method according to claim 1, wherein the synthetic silica-based deproteinizing agent is a synthetic silica gel or a synthetic silica colloid.

3. The method according to claim 2, wherein the synthetic silica-based deproteinizing agent is a synthetic silica gel having a particle size of approximately 1 to 50 μm, a specific surface area of approximately 100 to 1500 $m^2/g$, a pore volume of approximately 0.5 to 3 ml/g, and an average pore size of approximately 1 to 50 nm.

4. The method according to claim 1, wherein the aqueous solution containing avian egg yolk components including sialyl glycopeptide is a solution of chicken egg yolk or delipidated egg yolk in water.

5. The method according to claim 1, wherein the aqueous solution containing avian egg yolk components including sialyl glycopeptide is a solution from which an insoluble component has been removed beforehand.

6. The method according to claim 1, further comprising the step of recovering a fraction containing sialyl glycopeptide from the dissolved portion by column chromatography using a resin capable of separating sialyl glycopeptide.

7. The method according to claim 6, wherein the resin capable of separating sialyl glycopeptide is a reverse-phase resin, a normal-phase resin, an ion-exchange resin, a gel filtration resin, or a synthetic adsorbent resin.

8. The method according to claim 7, wherein the synthetic adsorbent resin is a styrene-divinylbenzene synthetic adsorbent resin, a modified styrene-divinylbenzene synthetic adsorbent resin, a methacrylic synthetic adsorbent resin, or a phenolic synthetic adsorbent resin.

9. The method according to claim 8, wherein the synthetic adsorbent resin is a modified styrene-divinylbenzene synthetic adsorbent resin.

## Patentansprüche

1. Verfahren zur Reinigung von Sialylglykopeptid, umfassend das Vermischen eines synthetischen Deproteinisierungsmittels auf Siliziumdioxidbasis mit einer wässrigen Lösung, die Vogeleidotterkomponenten einschließlich Sialylglykopeptid enthält, wodurch die Menge an Proteinen darin reduziert wird, und das Gewinnen eines gelösten Anteils aus dem Gemisch, wobei das Sialylglykopeptid in dem gelösten Anteil verbleibt.

2. Verfahren nach Anspruch 1, wobei das Deproteinisierungsmittel auf Siliziumdioxidbasis ein synthetisches Siliziumdioxidgel oder ein synthetisches Siliziumdioxidkolloid ist.

3. Verfahren nach Anspruch 2, wobei das Deproteinisierungsmittel auf Siliziumdioxidbasis ein synthetisches Siliziumdioxidgel mit einer Teilchengröße von etwa 1 bis 50 $\mu$m, einer spezifischen Oberfläche von etwa 100 bis 1500 $m^2/g$, einem Porenvolumen von etwa 0,5 bis 3 ml/g und einer durchschnittlichen Porengröße von etwa 1 bis 50 nm ist.

4. Verfahren nach Anspruch 1, wobei die wässrige Lösung, die Vogeleidotterkomponenten einschließlich Sialylglykopeptid enthält, eine Lösung von Hühnereidotter oder delipidiertem Eidotter in Wasser ist.

5. Verfahren nach Anspruch 1, wobei die wässrige Lösung, die Vogeleidotterkomponenten einschließlich Sialylglykopeptid enthält, eine Lösung ist, aus der zuvor eine unlösliche Komponente entfernt wurde.

6. Verfahren nach Anspruch 1, das weiterhin den Schritt der Gewinnung einer Fraktion, die Sialylglykopeptid enthält, aus dem gelösten Anteil durch Säulenchromatographie unter Verwendung eines Harzes, das Sialylglykopeptid abtrennen kann, umfasst.

7. Verfahren nach Anspruch 6, wobei das Harz, das Sialylglykopeptid abtrennen kann, ein Umkehrphasenharz, ein Normalphasenharz, ein Ionenaustauscherharz, ein Gelfiltrationsharz oder ein adsorbierendes synthetisches Harz ist.

8. Verfahren nach Anspruch 7, wobei das adsorbierende synthetische Harz ein adsorbierendes synthetisches Styrol-Divinylbenzol-Harz, ein adsorbierendes modifiziertes synthetisches Styrol-Divinylbenzol-Harz, ein adsorbierendes synthetisches Methacryl-Harz oder ein adsorbierendes synthetisches Phenol-Harz ist.

9. Verfahren nach Anspruch 8, wobei das adsorbierende synthetische Harz ein adsorbierendes modifiziertes synthetisches Styrol-Divinylbenzol-Harz ist.

## Revendications

1. Procédé de purification de sialyl glycopeptide, comprenant le mélange d'un agent de déprotéinisation à base de silice synthétique avec une solution aqueuse contenant des constituants de jaune d'œuf aviaire incluant du sialyl glycopeptide, réduisant ainsi la quantité des protéines à l'intérieur, et obtenant une portion dissoute du mélange, le sialyl glycopeptide demeurant dans la portion dissoute.

2. Procédé selon la revendication 1, l'agent de déprotéinisation à base de silice synthétique étant un gel de silice synthétique ou un colloïde de silice synthétique.

3. Procédé selon la revendication 2, l'agent de déprotéinisation à base de silice synthétique étant un gel de silice synthétique ayant une taille de particule d'approximativement 1 à 50 $\mu$m, une surface spécifique d'approximativement 100 à 1 500 $m^2/g$, un volume de pore d'approximativement 0,5 à 3 ml/g, et une taille moyenne de pore d'approximativement 1 à 50 nm.

4. Procédé selon la revendication 1, la solution aqueuse contenant les constituants de jaune d'œuf aviaire incluant du sialyl glycopeptide étant une solution de jaune d'œuf de poulet ou de jaune d'œuf délipidé dans de l'eau.

5. Procédé selon la revendication 1, la solution aqueuse contenant des constituants de jaune d'œuf aviaire incluant

du sialyl glycopeptide étant une solution à partir de laquelle un constituant insoluble a été retiré au préalable.

6. Procédé selon la revendication 1, comprenant en outre l'étape de récupération d'une fraction contenant du sialyl glycopeptide de la portion dissoute par chromatographie sur colonne en utilisant une résine capable de séparer le sialyl glycopeptide.

7. Procédé selon la revendication 6, la résine capable de séparer le sialyl glycopeptide étant une résine à phase inverse, une résine à phase normale, une résine échangeuse d'ions, une résine de filtration sur gel, ou une résine synthétique adsorbante.

8. Procédé selon la revendication 7, la résine synthétique adsorbante étant une résine synthétique adsorbante de styrène-divinylbenzène, une résine synthétique adsorbante de styrène-divinylbenzène modifiée, une résine synthétique adsorbante méthacrylique, ou une résine synthétique adsorbante phénolique.

9. Procédé selon la revendication 8, la résine synthétique adsorbante étant une résine synthétique adsorbante de styrène-divinylbenzène modifiée.

[Figure 1]

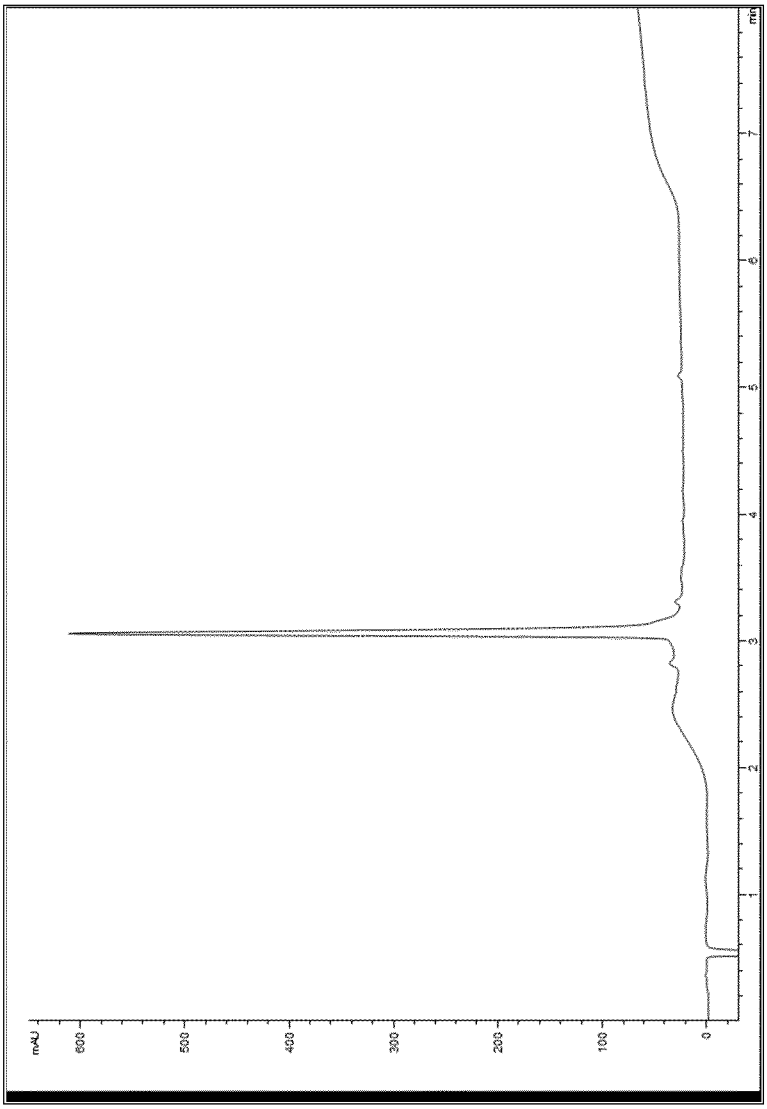

[Figure 2]

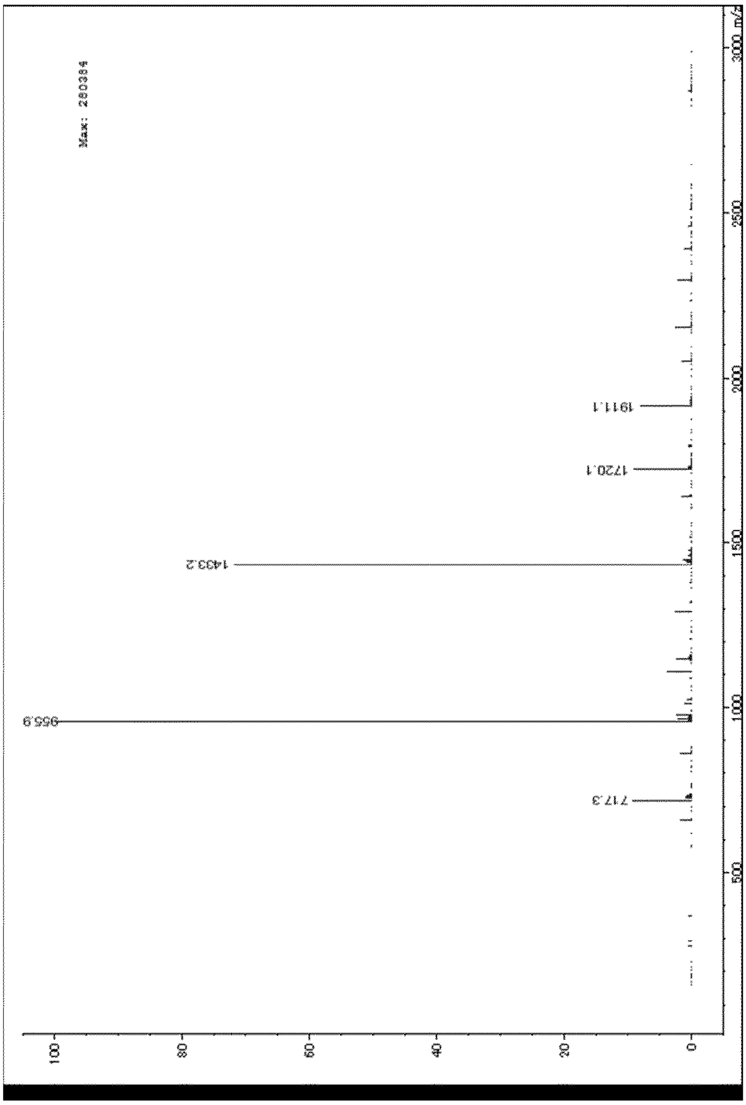

[Figure 3]

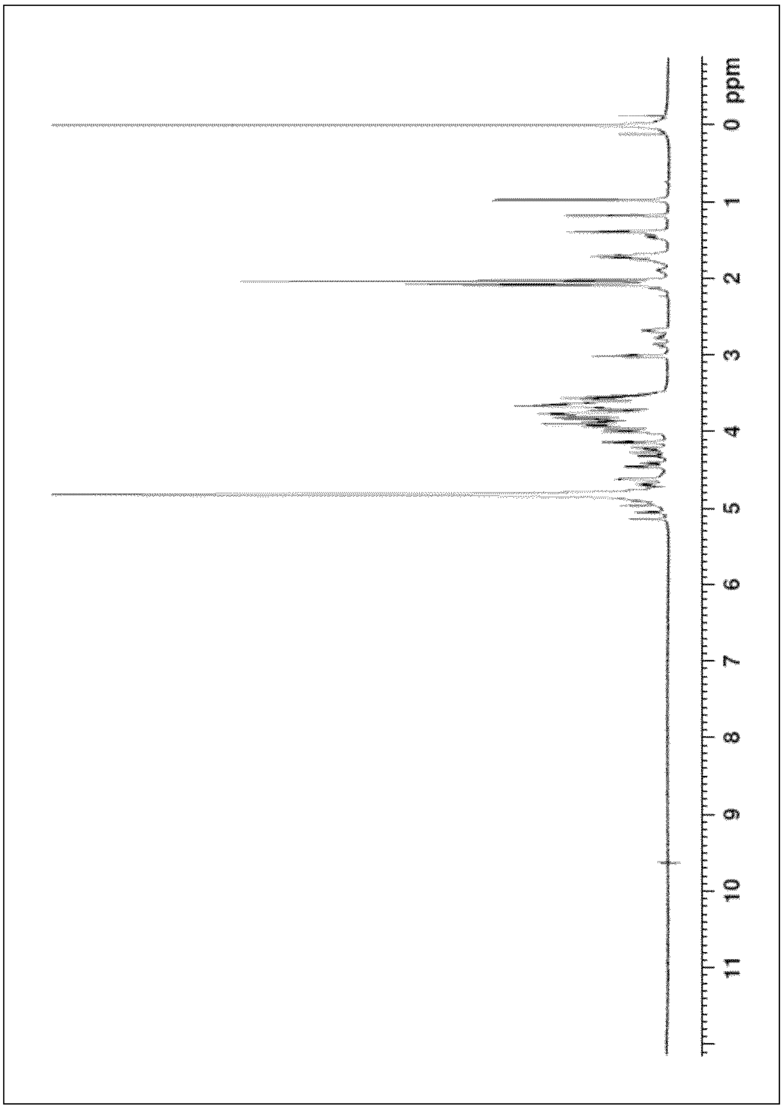

[Figure 4]

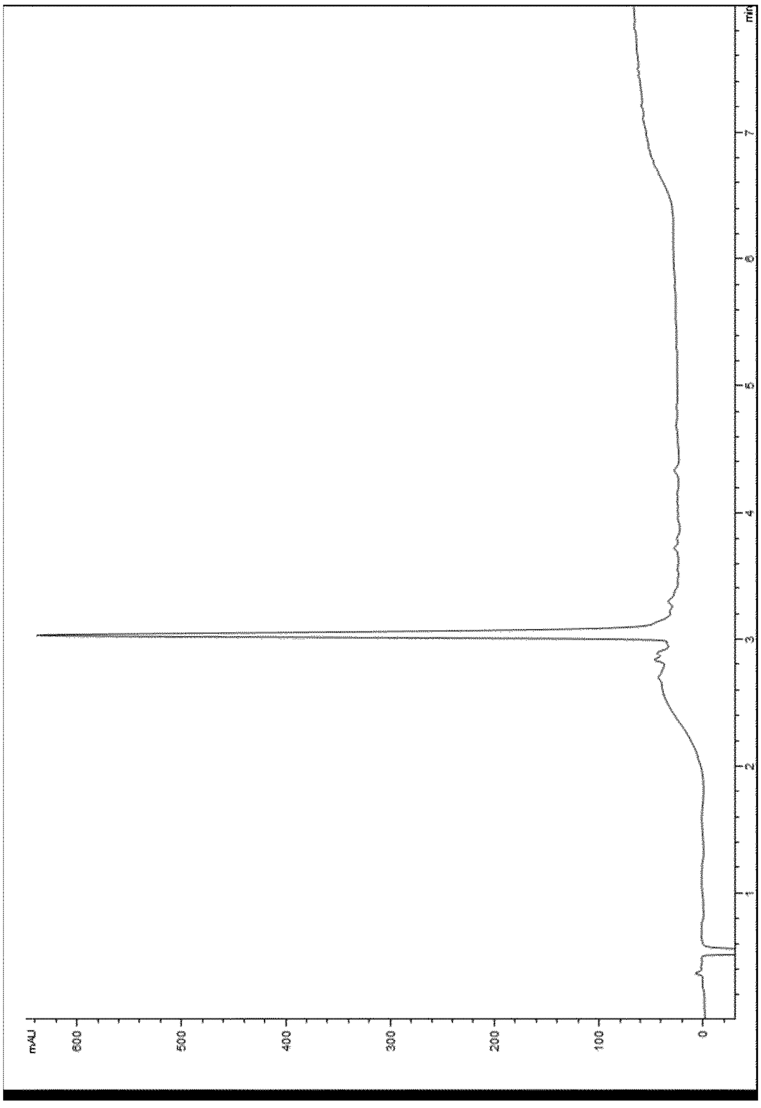

[Figure 5]

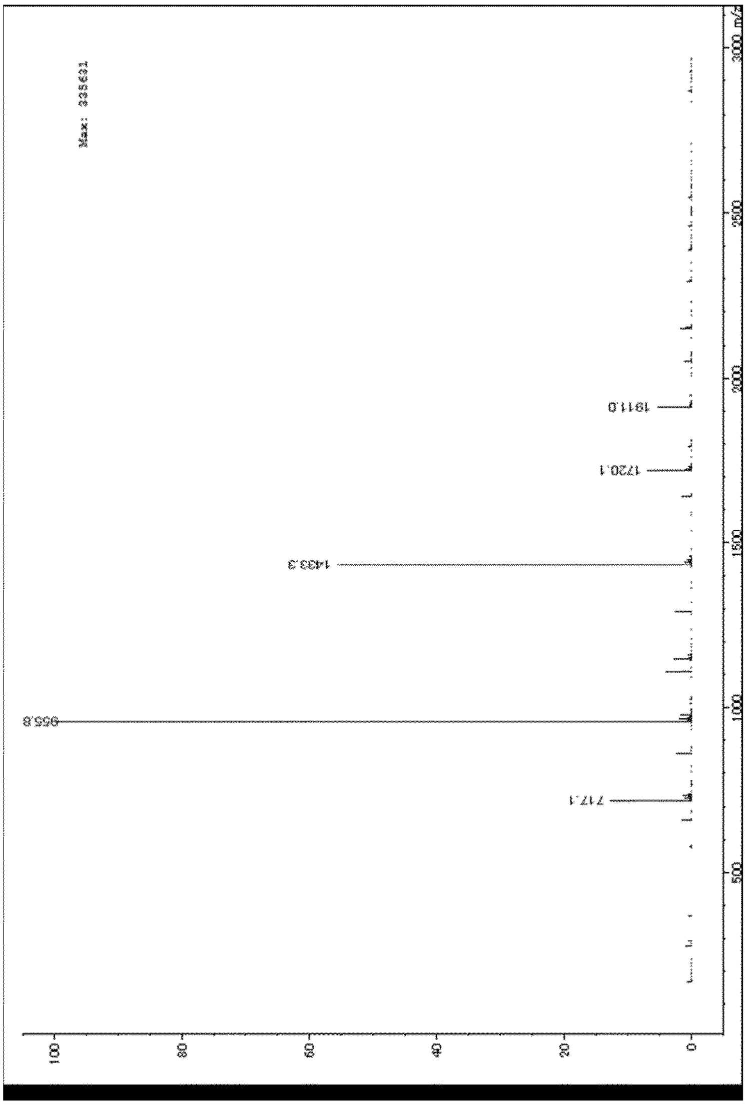

[Figure 6]

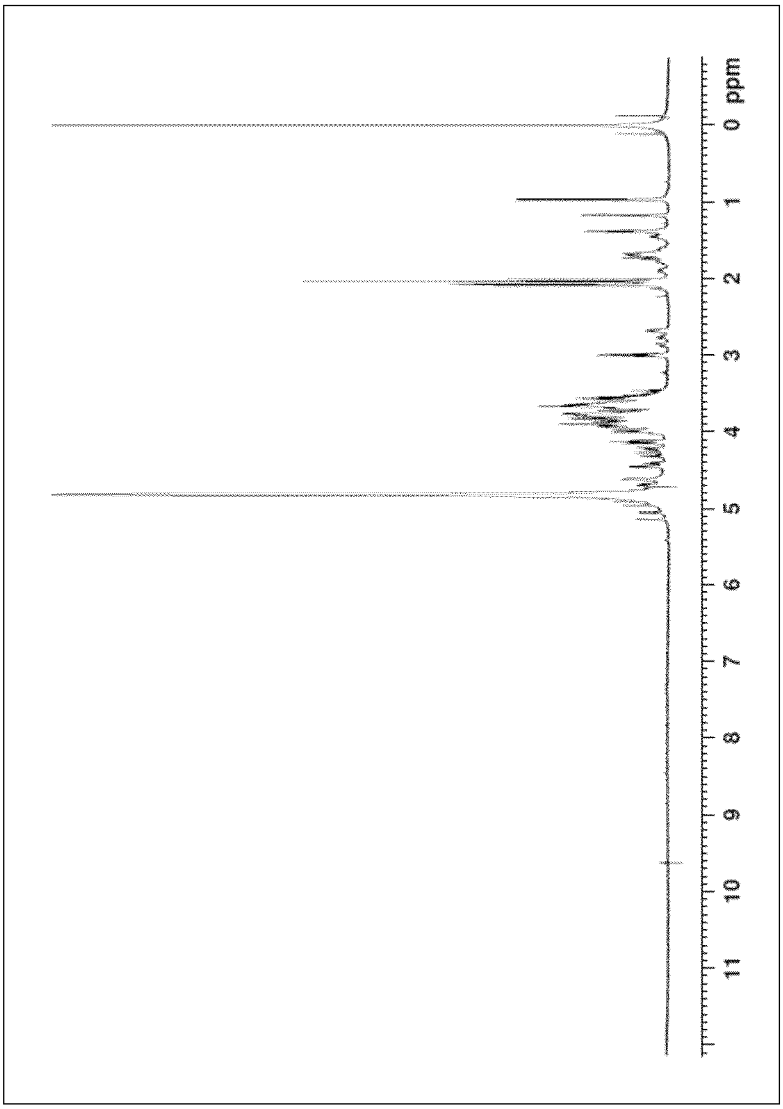

[Figure 7]

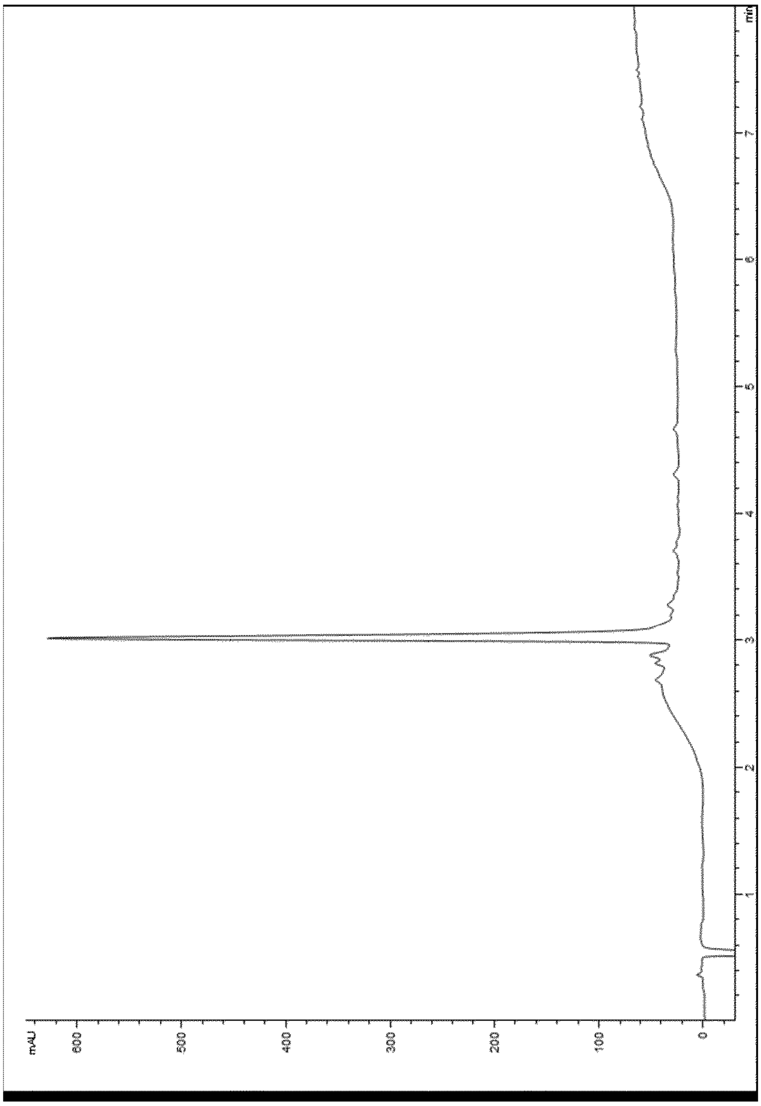

[Figure 8]

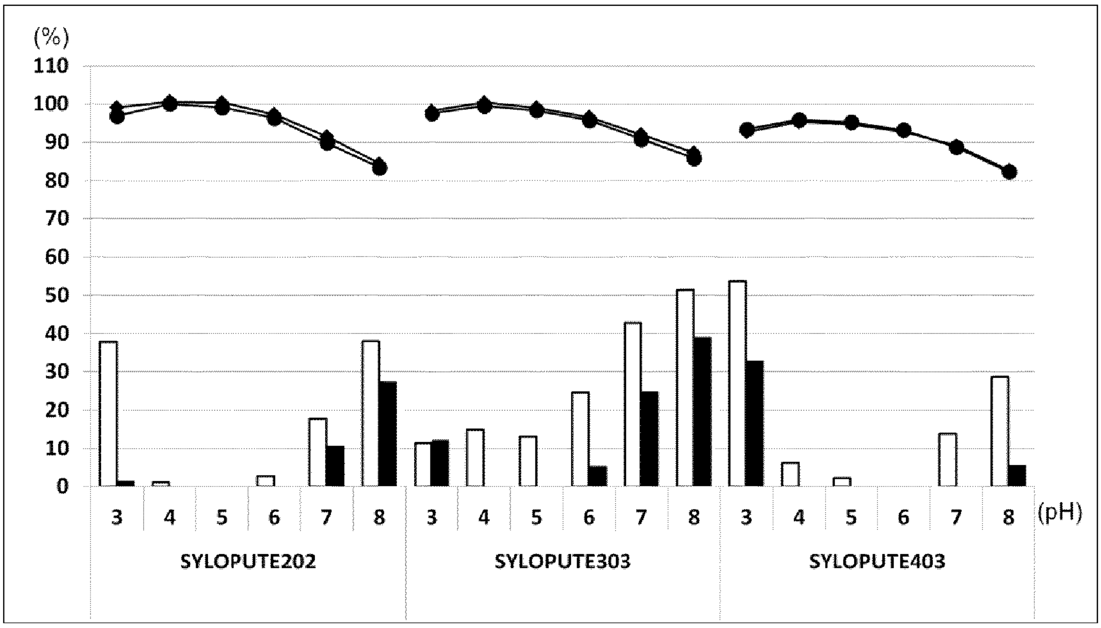


[Figure 9]

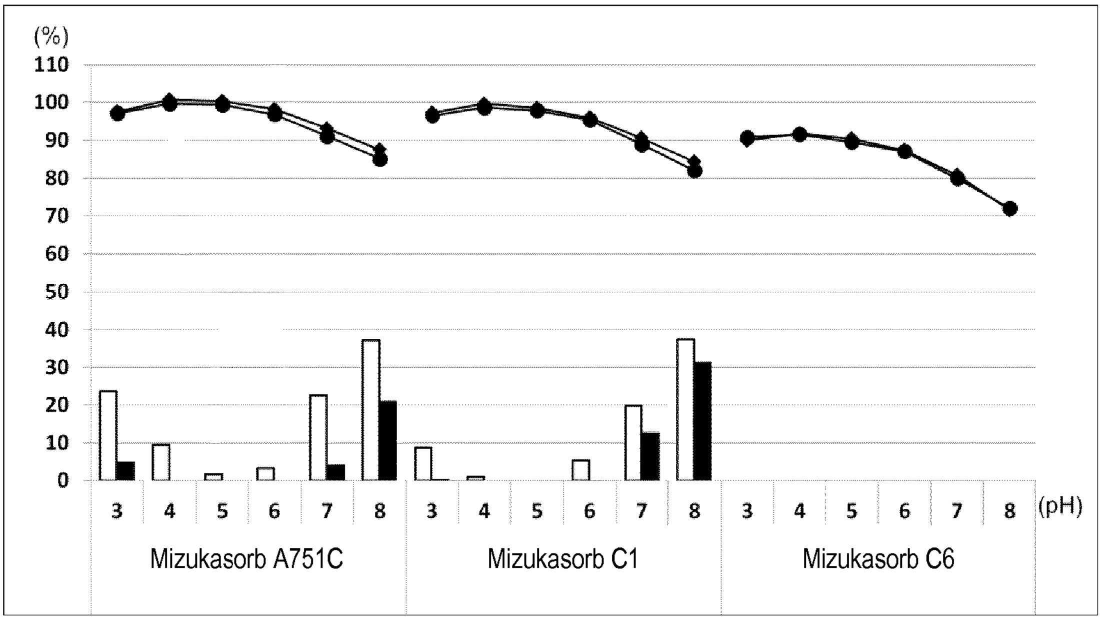

[Figure 10]

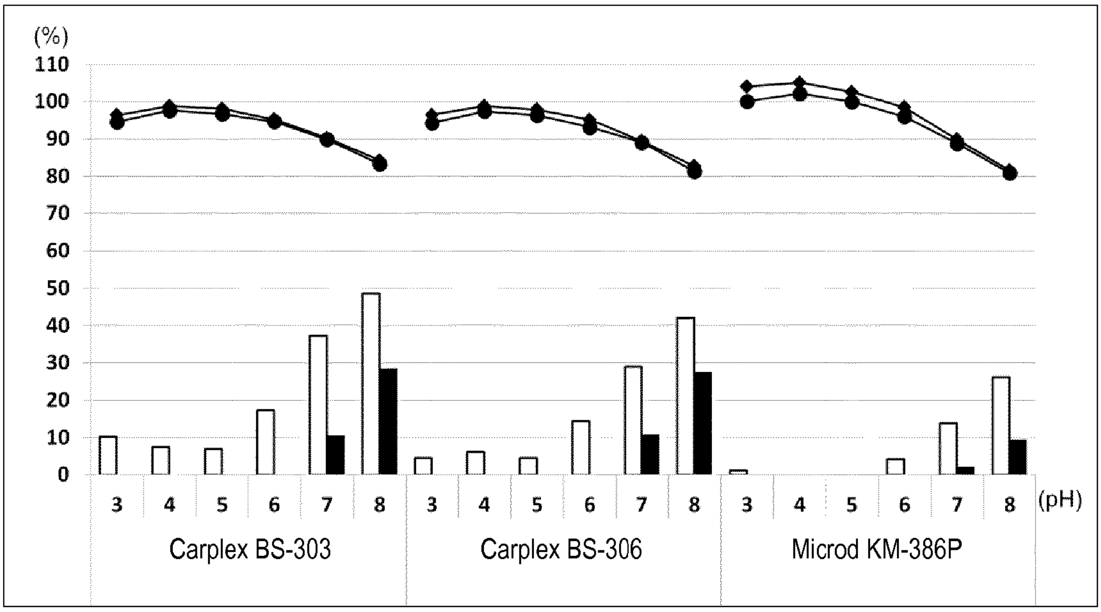


[Figure 11]

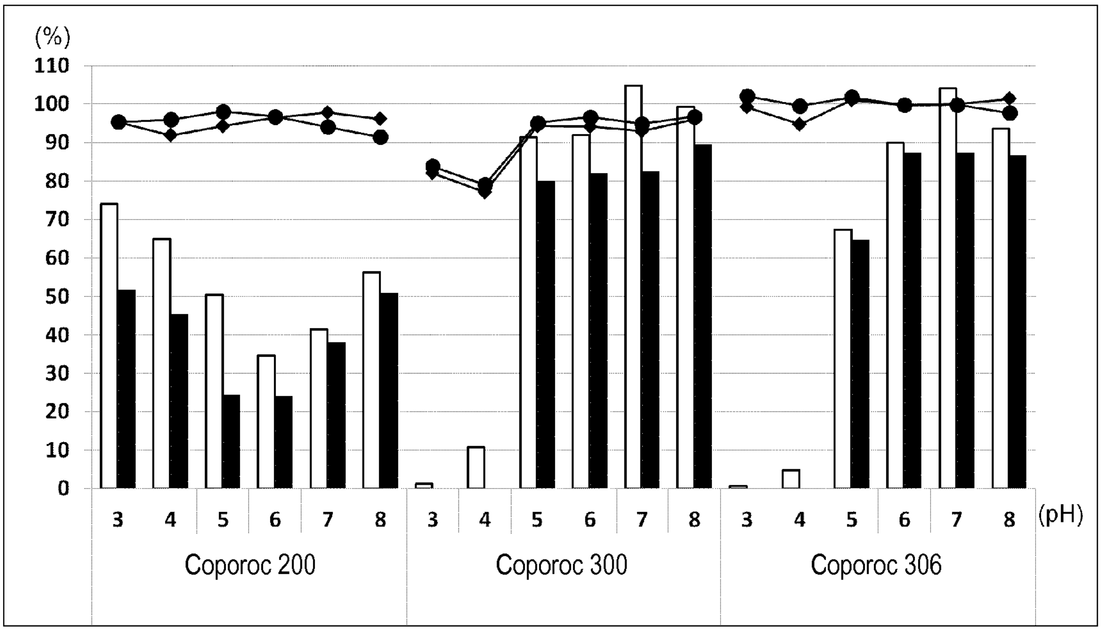

[Figure 12]

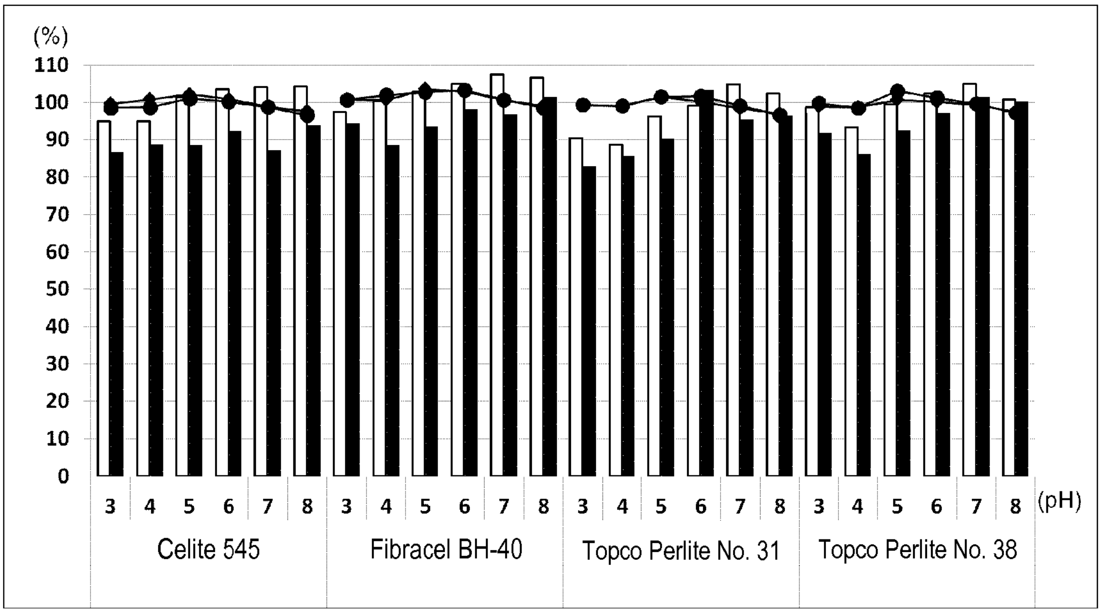


[Figure 13]

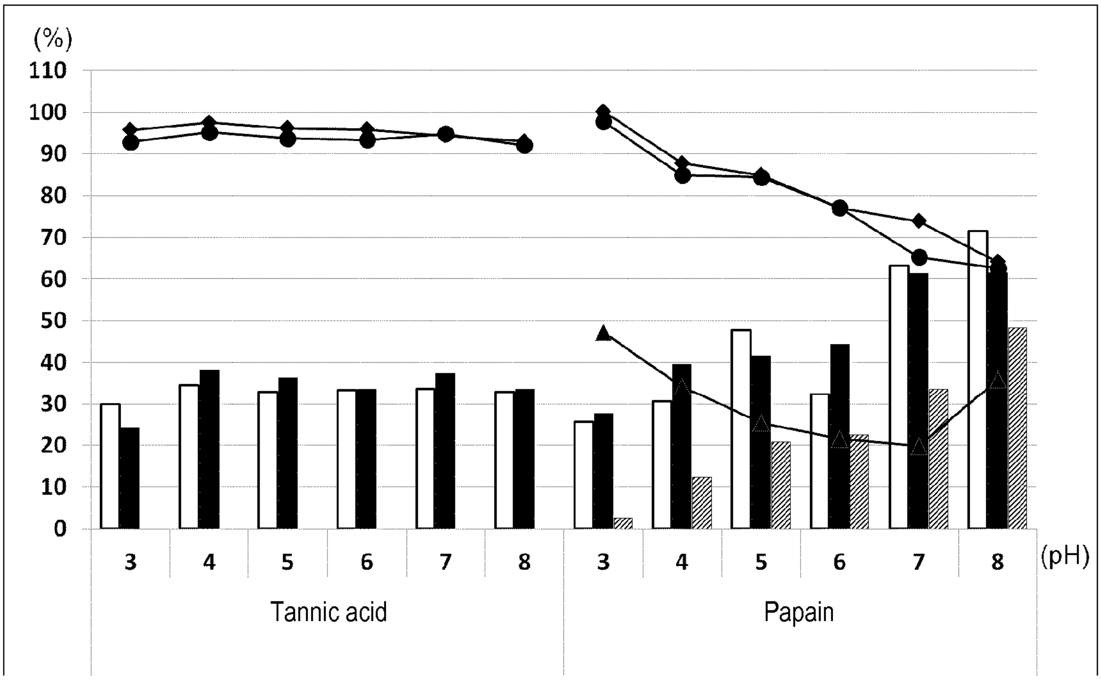

[Figure 14]

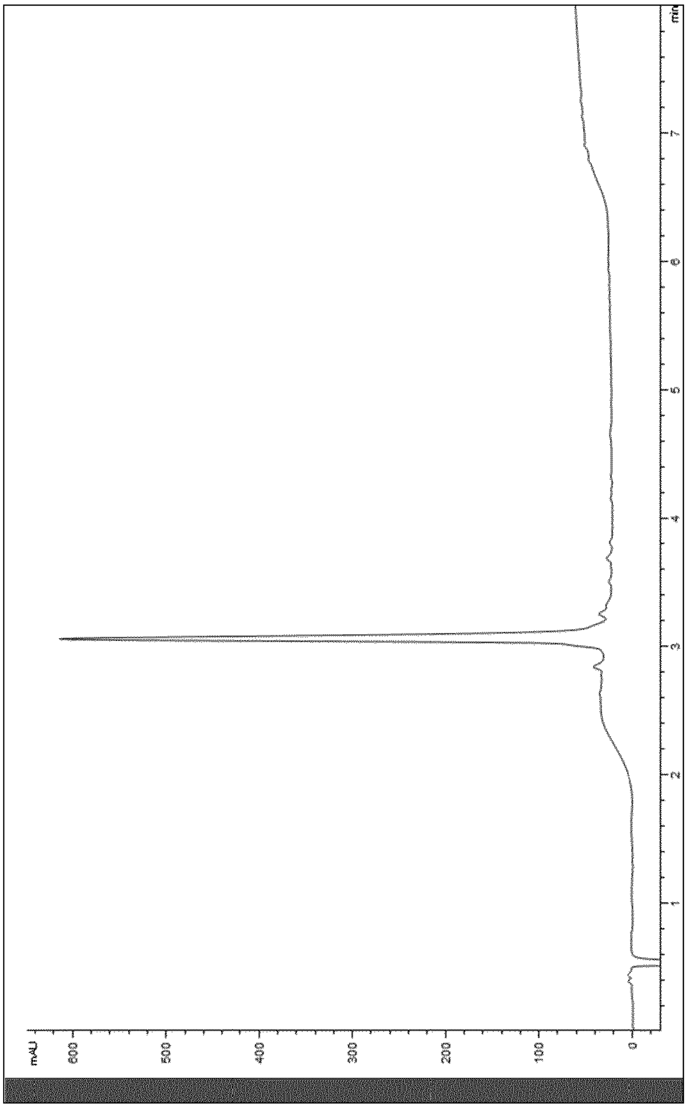

[Figure 15]

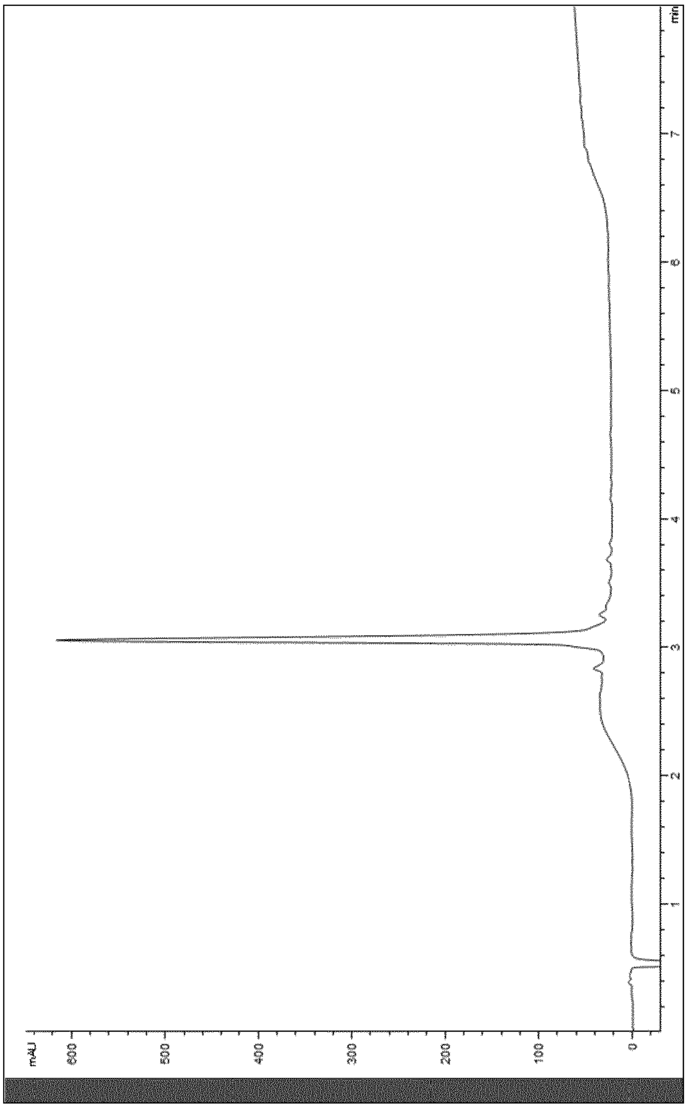

[Figure 16]

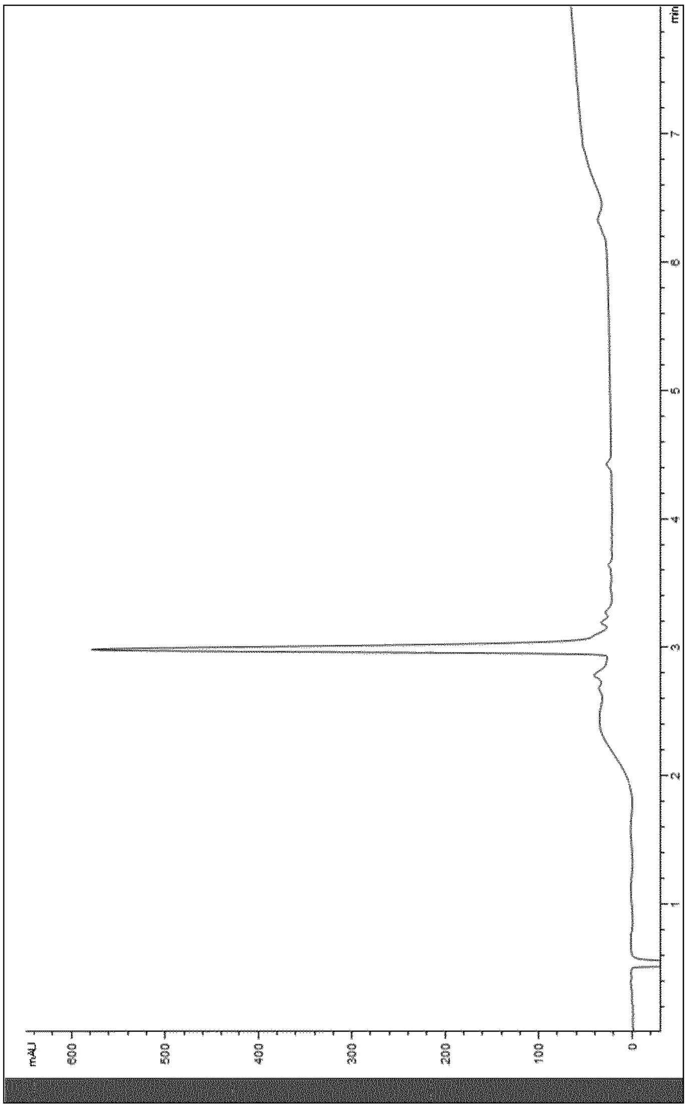

[Figure 17]

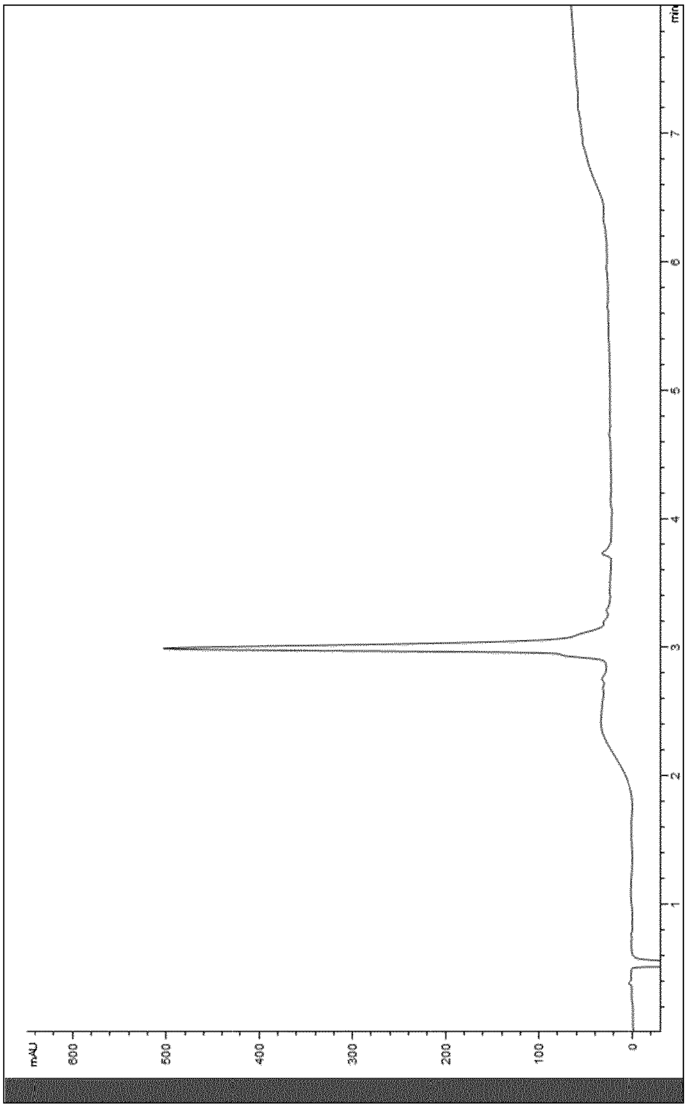

[Figure 18]

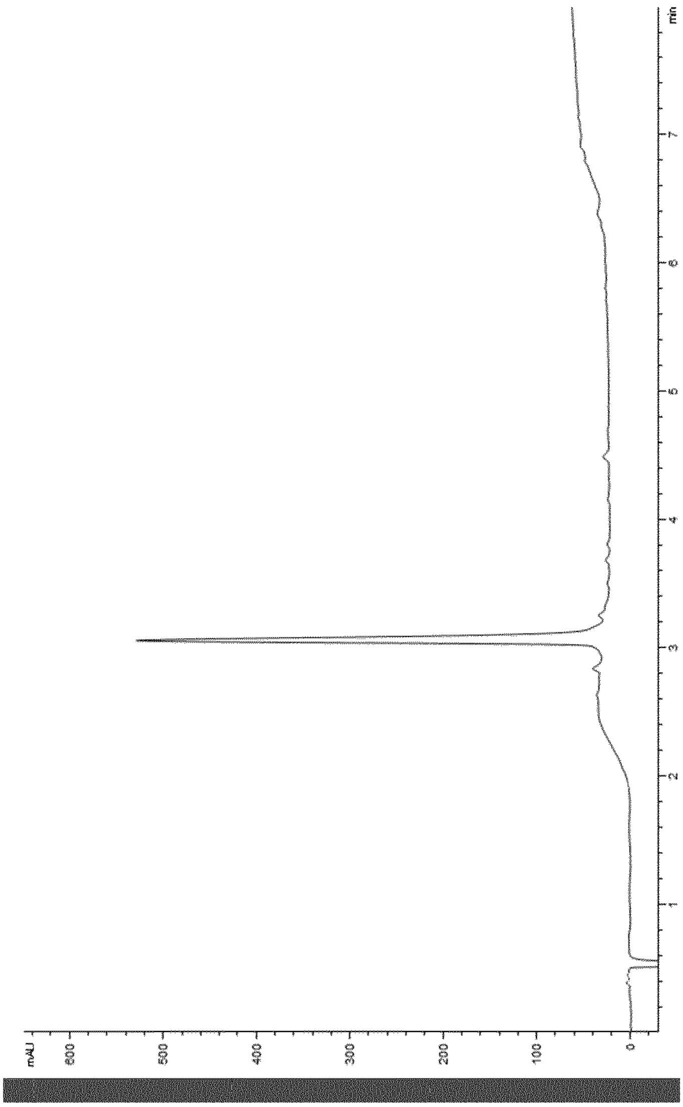

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011027868 A
- WO 9602255 A
- JP 2013136619 A


### Non-patent literature cited in the description


- **AKIRA SEKO et al.** *Biochimica et Biophysica Acta,* 1997, vol. 1335, 23-32
- **M.KOKETSU et al.** An efficient preparation and structural characterization of sialylglycopeptides from protease treated egg yolk. *JOURNAL OF CARBOHYDRATE CHEMISTRY,* vol. 14 (6
- **A.SEKO et al.** Occurrence of a sialylglycopeptide and free sialylglycans in hen's egg yolk. *BIOCHIMICA ET BIOPHYSICA ACTA,* vol. 1335 (1-2

SEQUENCE LISTING

<110> DAIICHISANKYO COMPANY, LIMITED

<120> A method for purification of glycopeptide

<130> FP1418

<150> JP2013- 136619
<151> 2013-06-28

<160> 1

<170> PatentIn version 3.5

<210> 1
<211> 6
<212> PRT
<213> Gallus gallus domesticus

<400> 1

```
Lys Val Ala Asn Lys Thr
1               5
```